# EUROPÄISCHE PATENTSCHRIFT

(11) **EP 2 439 183 B1**
(45) Veröffentlichungstag und Bekanntmachung des Hinweises auf die Patenterteilung: **25.12.2013**
(21) Anmeldenummer: 10186749.7
(22) Anmeldetag: 06.10.2010
(51) Int. Cl.: C04B 41/52, C04B 41/90, A61L 27/10, A61L 27/30, F41H 5/04, C23C 14/48

(54) **Monolithischer Keramikkörper mit Mischoxid-Randbereich und metallischer Oberfläche, Verfahren zu dessen Herstellung und dessen Verwendung**
Monolithic ceramic body with mixed oxide edge areas and metallic surface, method for producing same and use of same
Corps en céramique monolithique avec une zone superficielle en oxyde mixte et une surface métallique, son procédé de fabrication et d'utilisation

(43) Veröffentlichungstag der Anmeldung: 11.04.2012
(73) Patentinhaber: CeramOss GmbH, 5020 Salzburg (AT)
(72) Erfinder: Sorin, Lenz, 5020 Salzburg (AT); Mahringer, Christian, 5300 Hallwang (AT); Rübig, Günter, 4600 Wels (AT); Schreiner, Alexander, 81543 München (DE)
(74) Vertreter: Vogeser, Werner

(56) Entgegenhaltungen:
- EP-A2- 2 018 879
- DE-B3- 10 303 351

## Beschreibung

### Gebiet der Erfindung

Die vorliegende Erfindung betrifft einen monolithischen Keramikkörper, dessen Herstellung und Verwendung. Insbesondere betrifft die vorliegende Erfindung einen monolithischen Keramikkörper mit einem chemisch veränderten Randbereich aus einem Mischoxid, wobei der Randbereich eine metallische Oberfläche aufweist.
Der Keramikkörper findet insbesondere als Implantat Verwendung

### Hintergrund der Erfindung

Im Allgemeinen dienen Implantate als Ersatz für erkrankte oder verloren gegangene menschliche oder tierische anatomische Strukturen, wie Zähne, Gelenke, Extremitäten usw. Vorzugsweise sollten solche Implantate mit dem Knochen im Organismus verwachsen, um eine stabile, langfristig belastbare Verbindung zu bilden. Es gibt bereits sowohl Titanimplantate, als auch Keramikimplantate. Während Titanimplantate ihren festen Platz in der Medizin, Zahnmedizin und Tiermedizin mit über 30-jähriger Erfahrung eingenommen haben, werden Keramikimplantate erst seit kürzerer Zeit in der Implantologie verwendet. Sie haben sich aufgrund ihrer hervorragenden Biokompatibilität, Bioinertheit, Korrosionsfestigkeit sowie ihrer guten physikalischen Eigenschaften, vor allem durch den Einsatz als Implantate, sich in der Zahnmedizin etabliert, jedoch ossoeintegrieren sie nur schlecht bis gar nicht.

Titan hat die Vorteile, dass es sehr gut osseointegriert, d.h. mit dem Knochen verwächst, und nicht allergen ist. Die hohe Sauerstoffaffinität des Titans führt zu einer Bildung einer Titanoxidschicht auf der Titanimplantatoberfläche, was zu den vorteilhaften Eigenschaften führt. Knochen verwächst mit der Titanoxidschicht. Um die Kontaktfläche zwischen Implantat und Knochen so weit wie technisch möglich zu maximieren, wird die Oberfläche der Titanimplantate angeraut. Damit kann die Osseointegration noch weiter verbessert werden. Heute wird Titan z.B. für Zahnimplantate oder bei Hüftgelenken für Titanschalen, welche ein keramisches Insert aufnehmen benutzt, während in der Kieferorthopädie unter anderem Verankerungsimplantate aus Titan eingesetzt werden. Der Einsatz von Titan in der restaurativen Zahnheilkunde wurde durch Weiterentwicklungen der Gusstechnik sowie durch Nutzung von CAD/CAM und Funkenerosions-Verfahren zur Herstellung individueller Werkstücke möglich.

Titan hat jedoch folgende, speziell für die zahnärztliche Implantologie eminente Nachteile:

Es hat eine dunkle, fast schwarze Farbe und, falls es hochglanzpoliert ist, eine silbrige Farbe, wodurch die Ästhetik im Zahnhalsbereich sehr zu wünschen übrig lässt. Des weiteren lassen sich Titanimplantate in der Zahnmedizin an der Durchtrittsstelle aus dem Zahnfleisch nicht mit Ultraschallspitzen aus Metall reinigen, da das Material zerkratzt wird und Rauhigkeiten entstehen, welche vermehrte Zahnbelagbildung fördern. Zur Reinigung werden daher spezielle Kunststoffspitzen benötigt.

Oxidkeramik (Zirkoniumoxidkeramik, Alumina, Zirkonia-Alumina-Mischungen, etc.) ist ein extrem hartes, glattes und biologisch inertes Material, welches gegen Korrosion (Säure, Salze, Körperflüssigkeiten) absolut beständig ist. Des Weiteren ist es wegen seiner Härte extrem abriebfest, d.h. die Oberfläche kann nur mit Diamantwerkzeug verändert werden. Weiterhin bietet die weiße Farbe des Materials - zumindest für Zahnimplantate - in der Zahnmedizin herausragende ästhetische Vorteile. Diese Eigenschaften werden bereits in der Medizin genutzt, z.B. als Stents für Gefäße in der Kardiologie mit einer Oberfläche aus Keramik damit keine Anlagerungen von Körperzellen erfolgen. Bei den in der Zahnmedizin eingesetzten Keramik-Zahnimplantaten sind die oben erwähnten Vorteile nachteilig. Dadurch, dass das Material biologisch inert ist, wird das Implantat nicht oder nur ungenügend osseointegriert.

Um die Vorteile der beiden Werkstoffe, Oxidkeramiken und Titan, zu vereinen und die jeweiligen Nachteile soweit wie möglich zu eliminieren, wurden in letzter Zeit zwei Ansätze verfolgt: Implantate aus einem Titankörper mit einer (teilweisen) Keramikbeschichtung (Verblendung) und Implantate aus einem Keramikkörper mit einer Titan- oder Titanoxidbeschichtung. Bei dem ersten Ansatz werden diejenigen Bereiche des Titankörpers mit einer Keramikbeschichtung versehen, die nach der Implantation keinen Kontakt mit dem Knochen haben. Bei dem zweiten Ansatz werden die Bereiche des Keramikkörpers mit Titan oder Titanoxid beschichtet, welche sich nach der Implantation in Knochenkontakt befinden, um dort besser osseointegriert werden zu können. Die Bereiche des Implantates, welche nach der Implantation keinen Kontakt mit dem Knochen haben, bleiben unbeschichtet.

Aufgrund der materialspezifischen Eigenschaften des Titans, nämlich dessen niedriger Wärmeausdehnungskoeffizient, die extreme Affinität des Titans zu Luft und Sauerstoff sowie die Kristallgitterumwandlung bei 882°C, können die früher gebräuchlichen Metall-Keramik-Verbundsysteme (Metallgrundkörper mit Keramikoberfläche, Verblendkeramiken) nicht genutzt werden, denn es ist nicht möglich, eine Keramik metallisch zu "verblenden".

Durch eine Reaktion mit Keramikbestandteilen bildet sich bereits bei Temperaturen von 750-800°C eine oxidative Reaktionsschicht an der Oberfläche des Titankörpers. Bei Temperaturen von annähernd 1000°C, wie sie bei der Herstellung konventioneller Keramiken erreicht werden, würden die Oxidschichten extrem verstärkt und folglich der Verbund zur Keramikbeschichtung geschwächt werden. Überdies sind durch die Kristallgitterumwandlung Spannungen zu befürchten, die sich ebenfalls verbundschwächend auswirken können. Titan weist im Vergleich zu anderen Dentallegierungen einen besonders niedrigen thermischen Ausdehnungskoeffizienten auf. Die Wärmeausdehnungskoeffizienten von Keramik und Metall müssen jedoch aufeinander abgestimmt sein, um Risse und Abplatzungen der Keramik zu vermeiden, wie sie bei der Verblendung von Titan mit herkömmlichen Keramiken entstehen würden. Wie dem Fachmann bekannt, dehnen sich Metalle bei Hitze aus während Keramiken eine Sinterschrumpfung erfahren.

Lange Zeit konnten keine zufriedenstellenden Haftfestigkeitswerte von Titan-Keramik-Systemen erreicht werden. Der geringere Haftverbund zwischen Titan und Keramik wird sowohl auf die notwendige Anpassung des Wärmeausdehnungskoeffizienten, als auch auf die hohe Sauerstoffaffinität des Titans zurückgeführt, wodurch während des Brennvorgangs der Keramik ein ausgeprägtes Wachstum der Oxidschicht stattfindet. Die Sprödigkeit der Oxidschicht wird als primäre Ursache für die geringeren Verbundwerte angesehen.

Aus diesem Grunde wurden spezielle Binder (Haftvermittler) entwickelt, welche durch ihre reduzierenden Eigenschaften die Oxidation des Titans während des keramischen Brennvorgangs verhindern sollen (M. Kononen und J. Kivilahti, Bonding of low-fusing dental porcelain to commercially pure titanium, J Biomed Mater Res 1994, Band 28, Nr. 9, Seite 1027-35; U. Tesch, K. Päßler und E. Mann, Untersuchungen zum Titan-Keramik-Verbund, Dent Lab, 1993, Band 41, Seite 71-74). Um die hohe Oxidationsneigung des Titans zu kompensieren und dadurch die Haftfestigkeitswerte von Titan-Keramik-Systemen zu erhöhen, wurden spezielle Binder entwickelt, die auf der Titanoberfläche vorhandene Oxide auflösen und umschließen und durch ihre glasartige Beschaffenheit die Oberfläche gegen weitere Oxidation versiegeln (J. Tinschert, R. Marx und R. Gussone, Struktur von Keramiken für die Titanverblendung, Dtsch Zahnärztl Z, 1995, Band 50, Seite 31-4). Untersuchungen haben jedoch gezeigt, dass diese Vorgehensweise nur teilweise zum gewünschten Erfolg führte. Gilbert et al. berichteten über eine Verbesserung des Haftverbundes (J. L. Gilbert, D. A. Covey und E. P., Lautenschlager, Bond characteristics of porcelain fused to milled titanium, Dent Mater, 1994, Band 10, Nr. 2, Seite 134-140). Hung et al. hingegen konnten keine signifikante Verbesserung durch die Verwendung eines Binders feststellen (C.C. Hung, M. Okazaki und J. Takahashi, Effect of Bonding Agent on Strength of Pure Titanium-Pocelain System, J Dent Res, 1997, Band 76, Seite 60).

Ein Nachteil der Verwendung von Bindern liegt darin, dass ein weiterer Keramikbrand notwendig wird, der neben dem erhöhten Zeitaufwand vor allem eine zusätzliche thermische Belastung des Titans bedingt. Ebenso sind durch den Binder hervorgerufene ästhetische Nachteile nicht auszuschließen.

Unter der Zielsetzung, die Oxidation des Titans während des Brennvorgangs zu verringern, wurden Versuche unternommenen, den Keramikbrand unter Schutzgasatmosphäre durchzuführen (J. Geis-Gerstorfer; Ch. Schille und P. Klein, Geringere Oxidationsneigung unter Schutzgasatmosphäre, Dent Lab, 1994, Band 42, Seite 1235-1236), jedoch mit nur wenig Erfolg, da als Hauptsauerstofflieferant für die Oxidation des Titans vor allem die Keramikbestandteile verantwortlich gemacht werden (M. Kononen und J. Kivilahti, Fusing of dental ceramics to titanium, J Dent Res, 2001, Band 80, Nr. 3, Seite 848-854).

Einen weiteren Ansatz, die Haftfestigkeit in einem Titan-Keramik-System zu erhöhen, beschreibt die DE 10 2004 041 687 A1, gemäß der auf einen Körper aus Reintitan durch ein CVD-, PVD- oder Plasmaimmersions-lonenimplantations- und Abscheideverfahren eine Schicht aus Zirkoniumoxid aufgebracht wird, auf welche die für die Verblendung von Titan bestimmte Keramik ohne einen Binder aufgebrannt wird. Hierbei dient die Zirkoniumoxidschicht als Haftvermittler zwischen dem Titankörper und der aufgebrachten Keramikschicht.

Neuere Ansätze gehen davon aus, einen Keramikkörper mit Titan zu beschichten, da bekannt ist, dass mit Titan beschichtete Keramiken sehr gute Ergebnisse in Bezug auf die Osseointegration zeigen. Die WO 03/045268 A1 offenbart zum Beispiel ein einteiliges Zahnimplantat aus einem Keramikgrundkörper mit einer Titanbeschichtung.

Allerdings ist auch bekannt, dass die Haftfestigkeit zwischen der Titanbeschichtung und der Keramik Probleme aufwirft, wie aus der US 2001/0036530 A1 bekannt. Die US 2001/0036530 A1 beschreibt ein Implantat aus einem Verbundwerkstoff aus einer Zirkoniumoxidkeramik mit einer ersten Beschichtung aus Titan, einer zweiten Beschichtung ebenfalls aus Titan und optional einer dritten Beschichtung aus Hydroxylapatit. Dabei werden zur besseren Verankerung der ersten Beschichtung und der damit erwünschten besseren Haftfestigkeit Titanionen mittels lonenimplantation in die Keramik implantiert. Hierdurch konnte die Haftfestigkeit um 20% gegenüber bekannten Keramik-Titan-Verbundsystemen verbessert werden. Die offenbarten Titan-Keramik-Verbundsysteme weisen jedoch keine zufrieden stellenden Eigenschaften auf. Bei der Untersuchung der Haftfestigkeit konnten zwar keine Risse oder Sprünge beobachtet werden, jedoch lag die Haftfestigkeit mit durchschnittlich 67 MPa nur unwesentlich über der im Stand der Technik erreichten Haftfestigkeit von 41 MPa. Ein ähnlicher Ansatz wurde in der EP 2 018 879 A1 offenbart. Allerdings konnte auch hier keine zufrieden stellende Haftfestigkeit erreicht werden. Somit konnte letztendlich der Effekt, dass bei Ablösung der Schicht "blanke" Keramik zum Vorschein tritt, nicht verhindert werden. Dieser Effekt ist nicht nur, aber vor allem in der Implantologie nicht akzeptabel, da Materialversagen katastrophale Folgen nach sich zieht, weil Implantate versagensfrei Jahrzehnte bis optimalerweise lebenslang im Körper verweilen sollten.

Es gibt Anwendungen, nicht nur doch vor allem bei Implantaten, die eine sehr hohe Haftfestigkeit der Schicht erfordern. Derartige Anwendungen sind nicht nur dentale Anwendungen, sondern auch andere medizinische Anwendungen, wie bipolare Prothesen (Hemiendoprothesen) zur Behandlung von Schenkelhalsfrakturen. Die häufig eingesetzten Duokopfprothesen bestehen aus einem Kopf, einem Stiel und einer Pfanne, die z.B. aus Polyethylen besteht. Hierdurch ergibt sich das Problem, dass durch die hohe mechanische Beanspruchung die Polyethylenpfanne verschleißt. Dieser Verschleiß kann zum Verlust der Gleitfähigkeit des Gelenks führen. Vor allem führen die Abriebprodukte zu aseptischer Knochennekrose. Dies führt zum technischen Versagen der Duokopfprothese und zu Folgeschäden im gesunden Gewebe. Voranstehende Ausführungen bezüglich der Folgen der Abriebprodukte gelten auch für Metall-Metall Paarungen sowie Metall-Kunststoff Paarungen bei orthopädischen Gelenkprothesen.

Es besteht also Bedarf an Materialien für Implantate, die sowohl in chemischer als auch in mechanischer Hinsicht alle Anforderungen an die unterschiedlichsten Einsatzbereiche der Implantate erfüllen. Diese müssen dazu noch die Fähigkeit aufweisen zu osseointegrieren. Zudem besteht Bedarf an einem Verfahren, mit welchen diese Materialien einfach und kostengünstig in ausreichender Menge hergestellt werden können.

### Darstellung der Erfindung

Der Erfindung liegt daher die Aufgabe zugrunde, ein Material bereitzustellen, das biokompatibel ist, osseointegriert, und keine aseptischen Nekrosen durch seine Abriebprodukte verursacht. Weiterhin soll dieses Material die in allen Einsatzbereichen von Implantaten erforderlichen chemischen und mechanischen Eigenschaften aufweisen, sowie einfach herzustellen sein. Der Erfindung liegt ferner die Aufgabe zugrunde, die Schichthaftungsproblematik zu eliminieren und ein Verfahren bereitzustellen, das eine einfache Herstellung des Materials erlaubt. Phasengrenzenbildungen wie sie bei herkömmlichen Beschichtungen entstehen, sollten vermieden werden

Die der Erfindung zugrunde liegenden Aufgaben wurden durch die Bereitstellung eines monolithischen Keramikkörpers nach Anspruch 1, eines Verfahrens zur Herstellung eines monolithischen Keramikkörpers nach Anspruch 10 sowie seine Verwendung nach Anspruch 17 oder 18 gelöst. Bevorzugte Ausführungsformen sind den abhängigen Ansprüchen zu entnehmen. Die Erfinder haben erkannt, dass der Problemlösungsansatz bei neuen Werkstoffen im allgemeinen, und ganz speziell für die Implantologie, bei der Eliminierung der Schichthaftungsproblematik liegt, um eine jahrzehntelange Verweildauer und Funktionstüchtigkeit im Körper zu gewährleisten.

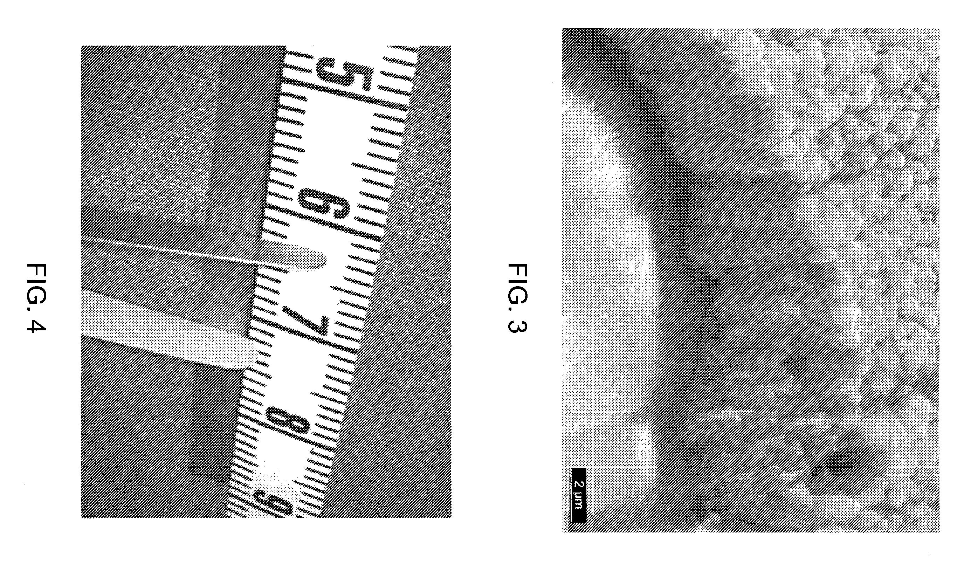

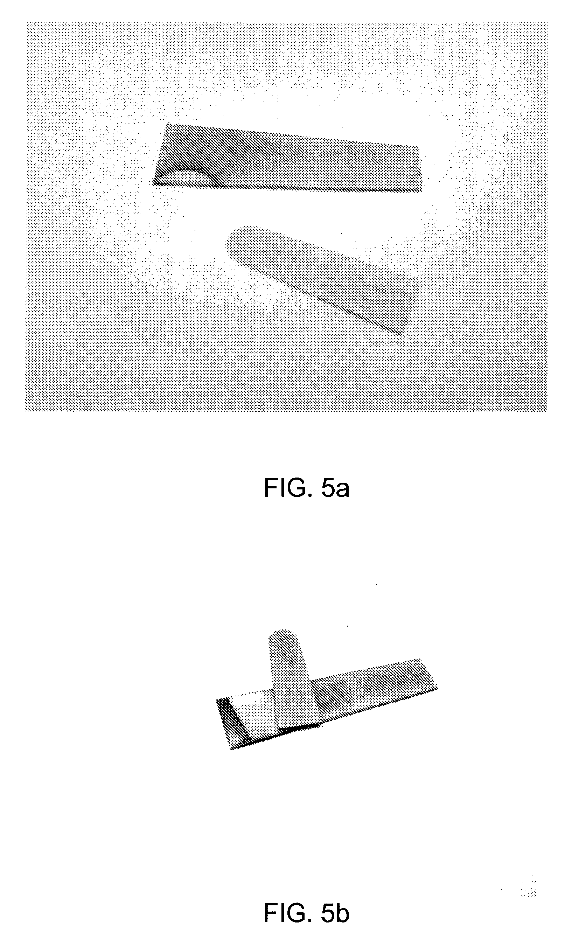

Mit der vorliegenden Erfindung werden außerdem folgende Vorteile und Wirkungen kombiniert erreicht:
- Schaffung eines osseointegrierenden monolithischen Keramikkörpers mit Mischoxid-Randbereich und metallischer Oberfläche mit einer der Knochenstruktur, bezüglich der Weichheit, soweit ähnlichen Struktur, dass Mikrofrakturen des knöchernen Implantatbettes bei Belastung weitestgehend verhindert werden können. Wie aus der Literatur bekannt und dort beschrieben, verursachen besonders harte Implantatmaterialien bei Belastungsspitzen unerwünschte knöcherne Mikrofrakturen des Knochens im Implantatbett - ein Problem, welches bisher noch keine Lösung fand, aber mit der vorliegenden Erfindung gelöst werden konnte.
- Erfindungsgemäße monolithische Keramikkörper mit Mischoxid-Randbereich und metallischer Oberfläche eliminieren die durch Mikrodefekte der Oberfläche (präformierte Sollbruchstelle) verursachten Schwachstellen des keramischen noch nicht erfindungsgemäß veränderten Grundkörpers. Nach erfindungsgemäßer Veränderung wird dieser resistenter gegen Schlag- und Stoßwirkung, und auch die Splitterneigung wird weitestgehend möglich eliminiert. ― dem Fachmann ist bekannt, dass herkömmliche Keramik sehr hart, aber auch sehr spröde ist, und bei Materialversagen in unzählige Bruchstücke zerspringt.
- Bei der Untersuchung erfindungsgemäßer monolithischer Keramikkörper in Form von Keramikplättchen mit einer Dicke von ca. 1 mm stellte sich heraus, dass diese Körper wesentlich biegsamer als nicht erfindungsgemäße Keramikkörper waren, und beim Bruch nicht so wie herkömmliche Keramikplättchen in unzählige kleine Teile zersplittern, sondern definiert mit einer Bruchstelle in zwei Stücke brechen (siehe Fig. 4, 5a und 5b).
- Erfindungsgemäße monolithische Keramikkörper sind wesentlich schlag- und druckresistenter durch Absorption und gleichmäßige Druckumverteilung, da der Mischoxid-Randbereich und die metallische Oberfläche wesentlich elastischer als Keramik sind und dadurch Mikrorisse zu verhindern vermögen. Dies bedeutet, dass die mechanische Überlastung bis zum Bruch wesentlich später erfolgt, da wie aus der Literatur bekannt, Mikrorisse an der Oberfläche von Keramik schnell durch die Keramik ziehen und diese zersplittern lassen. Derartiges Zersplittern kommt gerade im menschlichen Körper einer Katastrophe gleich, da all die Splitter entfernt werden müssen, was nicht immer vollständig gelingt. Konsekutiv verursachen die im menschlichen Körper verbliebenen Splitter persistierende Beschwerden. Dieses Problem wird durch die Verwendung erfindungsgemäßer Implantate soweit wie technisch machbar ausgeräumt und weitestgehend möglich vermieden.
- Erfindungsgemäße monolithische Keramikkörper verhalten sich an der Oberfläche wie Metalle. Damit kann eine weitergehende gewünschte Veränderung oder Bearbeitung der Oberfläche wie bereits aus der Metallverarbeitung bekannt kostengünstig erfolgen.

Der besondere Vorteil des erfindungsgemäßen monolithischen Keramikkörpers mit Mischoxid-Randbereich und metallischer Oberfläche besteht in der kumulierten Lösung vieler bisher noch nicht gelöster Probleme (oben aufgelistet). Darüber hinaus wird eine kumulierte wesentliche Verbesserung der Keramikeigenschaften gegenüber herkömmlichen Keramikkörpern erreicht. Dieses Ergebnis wird ohne Verlust der erwünschten und benötigten positiven Eigenschaften herkömmlicher Keramikkörper erzielt (Härte, Abriebfestigkeit, usw.), da die Herstellung der erfindungsgemäßen monolithischen Keramikkörper mit Mischoxid-Randbereich und metallischer Oberfläche im Niedrigtemperaturbereich stattfindet. Zusätzlich zu den dargestellten Problemlösungen wird bei der Verwendung des erfindungsgemäßen monolithischen Keramikkörpers mit Mischoxid-Randbereich und metallischer Oberfläche als Schutzpanzerung ein weiterer Vorteil erzielt. Die Oberfläche dient dabei als Gleitmittel (Abgleitmittel) bei z.B. auftreffenden Projektilen.

Der erfindungsgemäße Keramikkörper besteht aus dem Oxid eines ersten Metalls (I) mit einem Mischoxid-Randbereich (Metall I+II) sowie einer metallischen Oberfläche des Metalls (II). Der Mischoxid-Randbereich umfasst das Oxid des ersten Metalls (I) und das Oxid des weiteren Metalls (II), welches eine hohe Affinität zu Sauerstoff hat. Die Erfinder haben überraschend festgestellt, dass der Mischoxid-Randbereich einen kontinuierlichen, gleichmäßigen Konzentrationsgradienten des ersten Metalls (I), ausgehend von 100% im Kern bis zu 0% im Übergangsbereich zu der metallischen Oberfläche des Keramikkörpers, bezogen auf den Gesamtmetallgehalt (I+II), und einen kontinuierlichen, gleichmäßigen Konzentrationsgradienten des weiteren Metalls (II), ausgehend von 0% im Kern bis zu 100% im Übergangsbereich zu der metallischen Oberfläche des Keramikkörpers, bezogen auf den Gesamtmetallgehalt (I+II), aufweist. Die Sauerstoffkonzentration hingegen bleibt im Mischoxid-Randbereich konstant. Die Oberfläche des erfindungsgemäßen monolithischen Körpers ist metallisch (Metall II), und ist damit keine (metallische) Beschichtung.

Durch die erfindungsgemäße Herstellung entsteht ein monolithischer Keramikkörper mit Mischoxid-Randbereich und metallischer Oberfläche. Die bei einer Beschichtung klar erkennbaren Phasengrenzen sind bei dem erfindungsgemäßen Keramikkörper nicht existent, da es sich nicht um eine Beschichtung handelt, sondern um eine monolithische Struktur resultierend aus einer thermochemischen Reaktion.

Phasengrenzen (typisches Merkmal von Beschichtungen) sind weder im Übergangsbereich Metall (I) zum Mischoxid-Randbereich der Metalle (I+II), noch im Mischoxid-Randbereich selbst, noch im Übergangsbereich des Mischoxid-Randbereichs (Metall I+II) zur Metalloberfläche (Metall II) des Keramikkörpers feststellbar.

"Bereich" im Sinne der Erfindung bedeutet in Abgrenzung zu dem Begriff "Schicht", dass die chemische Zusammensetzung innerhalb des "Bereichs" variiert, auch innerhalb einer Atomlage des Bereichs. Hingegen zeichnet sich eine "Schicht" dadurch aus, dass sie Phasengrenzen aufweist und die ganze Schicht eine definierte chemische Zusammensetzung aufweist, die über die Schicht hinweg dieselbe ist.

"Keramik" im Sinne der Erfindung umfasst neben den Rohstoffen, die für die Herstellung keramischer Produkte verwendet werden, und ihrer Aufbereitung zur eigentlichen Keramik auch die aus Keramiken geformten und gebrannten Gegenstände selbst, die als Bauteile, Schutzpanzerungen für zivile und militärische Zwecke - bei Personen, Fahrzeugen, Gebäuden (Personen=Körperschutz, Gebäudepanzerung, Fahrzeugpanzerungen bei KFZ, Schiffen, U-Booten Flugzeugen, Raketen, usw.) - , Gebrauchs- und Ziergegenstände oder Werkzeuge verwendet werden.

"Metall (I)" und "Metall (II)" im Sinne der Erfindung bedeutet nicht die Oxidationsstufe der Metalle. Die Bezifferung (I) und (II) dient der Unterscheidung des Metalls, welches Bestandteil der Keramik ist, wobei hierfür die Bezeichnung "erstes Metall" oder "Metall (I)" verwendet wird. Bei dem Metall, welches zur Ausbildung des Mischoxid-Randbereichs verwendet wird, wird hierfür die Bezeichnung "weiteres Metall" oder "Metall (II)" verwendet. Die Begriffe "erstes Metall" und "Metall (I)" sowie "weiteres Metall" und "Metall (II)" werden synonym verwendet.

"Randbereich" im Sinne der Erfindung ist der Bereich des erfindungsgemäßen Keramikkörpers, der unterhalb seiner metallischen Oberfläche beginnt und in Richtung des Inneren des Keramikkörpers bis zu seinem Kern aus dem Oxid des ersten Metalls (I) verläuft.

"Randzone" im Sinne der Erfindung ist der Bereich des erfindungsgemäßen Keramikkörpers, der von der metallischen Oberfläche und dem darunter liegenden Randbereich gebildet wird.

"Unfertiger Keramikkörper" im Sinne der vorliegenden Anmeldung ist ein noch nicht erfindungsgemäß veränderter Keramikkörper.

"Randbereich des unfertigen Keramikkörpers" ist der Bereich des unfertigen Keramikkörpers, der ausgehend von dessen äußerer Oberfläche in Richtung des Inneren des unfertigen Keramikkörpers verläuft.

Die durch die Erfindung erreichten Vorteile sind insbesondere darin zu sehen, dass man bei dem erfindungsgemäßen Keramikkörper nicht mehr von einem Verbundwerkstoff, d.h. einem Keramikkörper mit einer Metallbeschichtung sprechen kann (da Phasengrenzen als Kennzeichen einer Beschichtung nicht mehr vorhanden sind). Es handelt sich stattdessen um einen monolithischen Keramikkörper mit Mischoxid-Randbereich und metallischer Oberfläche. Dementsprechend kann auch nicht mehr von einer "Schichthaftung" und Haftfestigkeit gesprochen werden. Vielmehr handelt es sich um einen Bereich des Keramikkörpers, der thermochemisch verändert wurde.

Bei herkömmlichen Verbundsystemen kommen drei Gruppen von Kräften zwischen dem Metall und der Verblendkeramik zum Tragen, die zum Verbund führen, nämlich mechanische, adhäsive und chemische Kräfte. Die mechanischen Kräfte entstehen durch das Aufschrumpfen einer Keramik auf das Metallgerüst während des Sinterprozesses. Für die Kräfte sind die Wärmeausdehnungskoeffizienten und die Retention, d.h. das mechanische Verzahnen der Verbundpartner miteinander, verantwortlich. Die zwischenmolekularen Anziehungskräfte (Van der Waals'sche Kräfte) sind für die Adhäsion zwischen den Verbundpartnern verantwortlich. Hierzu zählen insbesondere die Dipol-Wechselwirkungen und die Wasserstoffbrückenbindungen. Die Bildung eines Mischoxids führt zu der chemischen Kraft. Die Oberfläche der mit einer Keramik zu beschichtenden Metalle besteht, je nach Art des Metalls mehr oder weniger, nicht aus reinem Metall sondern aus Metalloxid. Diese Metalloxide sind retentiv und adhäsiv mit dem Metallgerüst verbunden. Der chemische Verbund zwischen dem Metallgerüst und der Keramik findet an der oxidierten Oberfläche des Metalls statt. Bei den keramischen Bränden kommt es zu gemeinsamen Bindungen zwischen der Metalloxidschicht und der Keramikgrundmasse. Es werden so genannte Sauerstoffbrücken gebildet. Entscheidend bei den herkömmlichen Verbundsystemen ist jedoch nicht nur, welche Kraft zu welchem Anteil wirkt, sondern auch, wie stark die Metalloxidschicht auf dem Metall haftet. Unabhängig davon, welche Kraft bei dem jeweiligen Verbund überwiegt, besteht das Verbundsystem aus vielen unterschiedlichen Schichten.

Die Erfinder haben bei dem erfindungsgemäßen monolithischen Keramikkörper mit Mischoxid-Randbereich und metallischer Oberfläche festgestellt, dass der Keramikkörper bis zur Oberfläche keinen Schichtaufbau (Phasengrenzen fehlen) aufweist. Es liegen nicht wie bei einer Beschichtung dünne Schichten mit gleicher chemischer Zusammensetzung und somit aufeinander liegende Schichten mit unterschiedlicher chemischer Zusammensetzung vor, welche aneinander haften, sondern man erhält ein komplexes System, in dem die Metallionen (II) mit dem Sauerstoff der Keramik (I) reagieren, so dass eine neue chemische Verbindung, bestehend aus Metallionen (I), Metallionen (II) und Sauerstoff, gebildet wird. Die Erfinder haben herausgefunden, dass zwischen den Sauerstoffatomen der Keramik (Oxid des Metalls I) als Festkörper und den Metallionen (II) eine (thermo-)chemische Reaktion stattfindet, wodurch sich ein Bereich ausbildet, bei dem der Randbereich des Keramikkörpers kontinuierlich chemisch verändert wird, bis hin zu einer äußeren Metalloberfläche, ohne dass es wie üblich nur zu einem "Einbau" der Metallionen (II) in das Gitter des Keramikmaterials kommt (hier sind Phasengrenzen vorhanden), wodurch das Gitter gestört und lonen aus dem Keramikgitter herausgeschlagen würden.

Vielmehr ist zu beobachten, dass die Metall (II)-Konzentration kontinuierlich, beginnend von 0% im Kern der Keramik bis 100% im Übergangsbereich zu der metallischen Oberfläche, bezogen auf den Gesamtmetallgehalt, zunimmt und die Metall (I)-Konzentration kontinuierlich beginnend von 100% im Kern der Keramik bis 0% im Übergangsbereich zu der metallischen Oberfläche, bezogen auf den Gesamtgehalt, abnimmt. Überraschend bleibt die Sauerstoffkonzentration im Mischoxid-Randbereich konstant. Folglich ändert sich die chemische Zusammensetzung des Keramikkörpers vom Körperinneren bis zu dessen Oberfläche, wobei im Randbereich ein Mischoxid aus Metall (I) und Metall (II) entsteht, welches schlussendlich in einer metallischen Oberfläche des Metalls (II) mit einer 100%-Konzentration des Metalls (II) endet.

Dies hat den Vorteil, dass es zu keiner Ausbildung von Schichten (Phasen, Phasengrenzen) kommt und daher zu keiner Begrenzung der Haftfestigkeit mehr. Alle Versuche, ein Materialversagen der Oberfläche des erfindungsgemäßen Monolithen herbei zu führen (Schichthaftungstests mit Superkleber) endeten im Versagen des Superklebers, ohne freiliegende Keramik. Der erfindungsgemäße Monolith blieb unbeschädigt. Die Schichthaftungsproblematik entfällt damit, genauso wie die Versuche, die Schichthaftung zu verbessern. Diese Problematik wurde erfindungsgemäß gelöst. Die Lösung der anderen bereits erwähnten Probleme wurde bereits erwähnt.

Damit handelt es sich konsekutiv auch nicht um eine Beschichtung. Es kann nicht mehr von Schichthaftung oder Haftfestigkeit gesprochen werden. Der Keramikkörper weist die vorteilhaften Eigenschaften einer metallbeschichteten Keramik auf und überwindet die Nachteile der Haftfestigkeit von herkömmlichen Metall-Keramik-Verbunden. Durch die chemische Veränderung des Randbereichs der Keramik wird ein Monolith geschaffen, mit einer untrennbaren chemischen Verbindung zwischen der Keramik (Oxid des Metalls I), dem Mischoxid-Randbereich auf Basis der Metalle (I) und (II) sowie der metallischen Oberfläche aus dem Metall (II).

Erfindungsgemäß ist die Keramik eine Oxidkeramik, bestehend aus dem Oxid eines Metalls (I), wobei das Metall (I) Zirkonium, Aluminium, Yttrium, Hafnium, Silizium, Magnesium, Cer, andere Metalloxide oder metallisches Glas oder Gemische davon umfasst. Bevorzugt ist das Metall (I) Zirkonium oder umfasst Zirkonium. Zirkoniumoxid und Alumina sind weiß und werden daher bevorzugt im Dentalbereich eingesetzt.

Der Keramikkörper kann vor der thermochemischen Ausbildung des Mischoxid-Randbereichs und vor dem Sintern vorgeformt werden. Dies bedeutet, dass eine grüne Keramik in eine gewünschte Form gebracht und anschließend gesintert wird. Dies hat den Vorteil, dass die grüne Keramik relativ weich und einfach zu formen ist, verglichen mit der harten Keramik nach dem Sintern. Dementsprechend können individualisierte bzw. maßgeschneiderte Implantate bei vergleichbar geringen Kosten, z.B. durch 3D-Rekonstruktion, hergestellt werden. Dies ermöglicht die Herstellung auch komplexer anatomischer Strukturen.

"Grüne Keramik" im Sinne dieser Erfindung bedeutet keramisches Material vor dem endgültigen Sinterprozess.

Die grüne Keramik kann gemäß dem Fachmann bekannten Verfahren hergestellt, geformt und bearbeitet werden, wie heiß isostatisches Pressen, Pressen, Drehen, Mahlen, Bohren, Beschleifen oder Spanen, etc. wobei die Verfahren manuell oder computergestützt numerisch gesteuert werden können.

Die vorgeformte Keramik kann vor oder nach dem Sintern mechanisch oder physikalisch behandelt werden, etwa um die Oberfläche zu vergrößern. Durch die vergrößerte Oberfläche wird die Osseointegration verbessert, wenn der erfindungsgemäße monolithische Keramikkörper mit Mischoxid-Randbereich und metallischer Oberfläche als Implantat verwendet wird. Die chemische, mechanische oder physikalische Behandlung wird bevorzugt an der grünen Keramik vorgenommen, da hier durch das weiche Material die Behandlung schneller, einfacher und billiger erfolgen kann als nach dem Sintern, kann aber auch nach dem Sintern erfolgen.

"Mechanische Behandlung" im Sinne der Erfindung umfasst insbesondere Schleifen, Sandstrahlen oder Bestrahlen mit einem Wasserstrahl, sowie alle weiteren dem Fachmann bekannten Verfahren. "Physikalische Behandlung" im Sinne der Erfindung umfasst insbesondere Bestrahlen mit einem Laserstrahl, sowie alle weiteren dem Fachmann bekannten Verfahren.

Darüber hinaus kann die grüne Keramik auch chemisch behandelt werden, z.B. Ätzen mit einer Säure oder einem Säuregemisch. Die Säure oder das Säuregemisch kann unter Phosphorsäure, Schwefelsäure, Chlorwasserstoffsäure, Fluorwasserstoffsäure, Salpetersäure, Salpetersäure/ Chlorwasserstoffsäure-Gemisch, wie Königswasser, oder Chlorwasserstoffsäure/Schwefelsäure-Gemisch ausgewählt werden. Dasselbe gilt auch für gesinterte Keramik, die mit geeigneten Säuren oder Säuregemisch behandelt werden kann (alle dem Fachmann bekannten und geeigneten Verfahren).

Das Metall (II) zur Ausbildung des Mischoxid-Randbereichs auf Basis der Metalle (I) und (II) und der metallischen Oberfläche aus dem Metall (II) ist erfindungsgemäß ein Metall mit hoher Sauerstoffaffinität und ist unter Titan, Niob, Tantal sowie Verbindungen und Legierungen davon ausgewählt. Weitere Sauerstoff affine Metalle sind nicht ausgeschlossen.

Bevorzugt ist das Metall (II) elementares Titan, eine Titanverbindung oder eine Titanlegierung. In einigen Ausführungsformen kann die Titanverbindung eine Verbindung aus Titan mit Elementen der 14. (z.B. C, Si, Ge, Sn, Pb), 15. (z.B. N, P, As, Sb, Bi) oder 16. (z.B. O, S, Se, Te, Po) Gruppe des Periodensystems oder ein Gemisch davon sein. Besonders bevorzugt ist als Metall (II) elementares Titan, wobei 100%-tiges Reintitan ganz besonders bevorzugt ist.

Die Dicke des Mischoxid-Randbereichs wird zum einen durch die Eindringtiefe der Metallionen (II) beim erfindungsgemäßen Implantieren, zum anderen durch deren Diffusion sowie durch die thermochemische Reaktion im Keramikkörper bestimmt. Hier findet die gewünschte chemische Reaktion statt, was das wesentliche Unterscheidungsmerkmal zur herkömmlichen lonenimplantation darstellt, bei der es nur zu einem "Einbau" von Metallionen in das Gitter des Keramikmaterials kommt (Phasengrenzen sind vorhanden). Der reaktive Randbereich hat im Mittel eine Dicke von etwa 700 Atomlagen, was etwa 140 Nanometer entspricht. Erfindungsgemäß beträgt die Dicke mindestens 500 Atomlagen, kann aber auch weniger betragen, jedoch nur soweit weniger, dass keine Schwächung des Monolithen erfolgt. Bevorzugt werden mindestens 700 Atomlagen und besonders bevorzugt mehr als 700 Atomlagen.

Ein Randbereich mit einer Dicke größer als 700 Atomlagen ist schwierig herzustellen, vor allem besonders kostenintensiv und bringt keinen ersichtlichen Vorteil oder weitere Verbesserungen hinsichtlich der Anwendungsbereiche des monolithischen Keramikkörpers mit Mischoxid-Randbereich und metallischer Oberfläche und hinsichtlich der erzielten Materialvorteile.

Die Dicke der Randzone von der äußeren Metalloberfläche aus dem Metall (II) bis zum Metall (I) im Inneren des Keramikkörpers (Mischoxid-Randbereich auf Basis der Metalle (I)+(II) inkludiert) beträgt im Schnitt 6-8 Mikrometer. Diese Randzone kann eine Dicke von 0,05 Mikrometer (geringere Dicke wird ausdrücklich nicht ausgeschlossen), bis hin zu mehreren Millimetern (größere Dicke wird ausdrücklich nicht ausgeschlossen) betragen. Bevorzugt werden Dicken zwischen 0,05 und 80 Mikrometer, ganz besonders bevorzugt zwischen 5 und 20 Mikrometer

In weiteren erfindungsgemäßen Ausführungsformen kann der Keramikkörper, falls erforderlich, mit einer oder mehreren Beschichtungen aus dem Metall (II) und/oder einer oder mehreren Beschichtungen aus einem biokompatiblen und /oder bioaktiven Material versehen werden, insbesondere mit einer mikroporösen Titanbeschichtung.

Eine Möglichkeit der "knochenfreundlichen" Oberflächengestaltung ist bisher die Beschichtung mit Calciumphosphat (auch Beta-Tricalciumphosphat, etc.), das als bioaktiv (osseoaktiv) gilt, das heißt es fördert die Entstehung von Knochengewebe und stellt diesem anorganische Bestandteile für das Wachstum zur Verfügung. Breite Anwendung in der Implantologie hat die Hydroxylapatitbeschichtung gefunden. Die chemische Zusammensetzung des Beschichtungsmaterials, seine Haftfestigkeit an der Trägersubstanz, die Beschichtungsdicke sowie resorptive Vorgänge innerhalb der Beschichtung beeinflussen die Reaktion des Knochengewebes und somit die klinische Anwendbarkeit beschichteter Implantate.

Das biokompatible/bioaktive Material kann ferner unter Antibiotika, Wachstumsfaktoren, Peptiden, Fibronektin und entzündungshemmenden Stoffen ausgewählt werden. Weitere dem Fachmann bekannte biokompatible/bioaktive Materialien können Anwendung finden, und werden ausdrücklich nicht ausgeschlossen.

Als Antibiotika können beispielhaft Amikacin, Gentamicin, Kanamycin, Neomycin, Netilmicin, Paromomycin, Streptomycin, Tobramycin, Cephalosporins, Fluoroquinolon-Antibiotika, Azithromycin, Erythromycin, Clarithromycin, Dirithromycin, Roxithromycin, Telithromycin, Penicilline, Ampicillin, Sulfonamide, Tetracycline, Clindamycin, Metronidazol und Vancomycin, etc. genannt werden.

Als Wachstumsfaktoren können beispielhaft Transforming growth factor beta (TGF-β), Granulocyte Colony Stimulating Factor (G-CSF), Granulocyte-Macrophage Colony Stimulating Factor (GM-CSF), Nervenwachstumsfaktor (NGF), Neurotrophins, Platelet Derived Growth Factor (PDGF), Erythropoietin (EPO), Thrombopoietin (TPO), Myostatin (GDF-8), Growth Differentiation Factor-9 (GDF9), Acidic Fibroblast Growth Factor (aFGF oder FGF-1 ), Basic Fibroblast Growth Factor (bFGF oder FGF-2), Epidermal Growth Factor (EGF), Hepatocyte Growth Factor (HGF), insulinähnliche Wachstumsfaktoren (IGFs) und Bone Morphogenetic Proteins (BMPs), etc. genannt werden.

Als entzündungshemmende Stoffe können beispielhaft Glucocorticoide, Corticosteroide und nicht Steroide entzündungshemmende Medikamente (z.B. Ibuprofen, Aspirin und Naproxen, etc.) genannt werden.

Ein Peptid kann beispielsweise ein bioaktives Peptid wie die RGD-Sequenz sein.

In einer besonderen erfindungsgemäßen Ausführungsform umfasst das biokompatible Material eine bioaktive Oberflächenbeschichtung aus osteochondralen/ossären Stammzellen oder chondralen Stammzellen oder einem Gemisch davon. Die Stammzellen verbessern die Osseointegration des damit beschichteten monolithischen Keramikkörpers mit Mischoxid-Randbereich und metallischer Oberfläche.

Als besonders vorteilhaft hat sich herausgestellt, wenn die Oberfläche des monolithischen Keramikkörpers mit Mischoxid-Randbereich und metallischer Oberfläche vor der Beschichtung mit einem biokompatiblen Material chemisch, mechanisch oder physikalisch zu deren Vergrößerung behandelt wird.

Mit dem erfindungsgemäßen Verfahren können in einfacher Weise monolithische Keramikkörper mit Mischoxid-Randbereich und metallischer Oberfläche hergestellt werden.

Das erfindungsgemäße Verfahren zur Herstellung eines monolithischen Keramikkörpers mit einem Mischoxid-Randbereich und metallischer Oberfläche umfasst folgende Schritte, die in einer thermochemischen Reaktionskammer an einem unfertigen Keramikkörper mit einem Randbereich durchgeführt werden:
(a) Evakuierung der Reaktionskammer auf einen Unterdruck, der 10⁻³ mbar oder weniger beträgt,
(b) Aktivierung des Randbereichs des unfertigen Keramikkörpers
(c) Starten der thermochemischen Behandlung des Randbereichs des unfertigen Keramikkörpers.

In Schritt (a) ist ein Hochvakuum zwischen 10⁻³ mbar und 10⁻⁷ mbar bevorzugt. Besonders bevorzugt ist ein Vakuum, das dem im Weltraum vorzufindenden Vakuum möglichst nahe kommt.

Die Evakuierung erfolgt am besten Stunden vor dem Prozessstart, um die Reaktionskammer sowohl von störenden Komponenten und Verunreinigungen zu befreien, als auch um überhaupt die beabsichtigte thermochemische Reaktion an einem Festkörper zu ermöglichen. Ein weiterer Vorteil des Hochvakuums liegt darin, dass die freie Weglänge der Metallionen (II) relativ hoch ist, bis es zu Stößen mit anderen Teilchen, wie Verunreinigungen oder Inertgasatomen bzw. -ionen, kommt, wodurch die Metallionen (II) Energie verlieren können. Bedingt durch das Hochvakuum erfolgt kein Energieverlust der Titanionen durch Reibung in ihrer Bewegung zur Keramik.

Erfindungswesentlich ist, dass die Reaktionskammer im Wesentlichen frei von Verbindungen, insbesondere Sauerstoff ist, mit denen die weiteren Metallionen (II) reagieren können. Als Verbindungen im Sinne der Erfindung werden chemische Verbindungen und Atome/Ionen verstanden.

Befinden sich derartige Verbindungen in der Reaktionskammer, können die hochenergetischen Metallionen (II) mit diesen Verbindungen, insbesondere Sauerstoff, reagieren, was zur Bildung unerwünschter Verbindungen, wie Titanoxid, führt, und stehen nicht mehr zur Ausbildung des Mischoxid-Randbereichs zur Verfügung. Die gebildeten Verbindungen können, wenn deren Energie noch ausreichend ist, ferner in den Randbereich des Keramikkörpers implantiert werden, was zu den mit der herkömmlichen lonenimplantation einhergehenden Nachteilen, wie die Störung des Keramikgitters, führen kann. Ferner können sich die unerwünschten Verbindungen als Oberflächenbeschichtung auf dem Keramikkörper absetzen und so eine störende Schicht aufbauen, die wiederum die Ausbildung des Mischoxid-Randbereichs zu verhindern vermag.

Es muss daher sichergestellt werden, dass die Metallionen (II) ungehindert, d.h. ohne auf dem Weg zwischen Target und Keramikkörper zu reagieren, auf den Keramikkörper auftreffen, um mit diesem thermochemisch und gleichmäßig reagieren zu können.

In Schritt (b) des erfindungsgemäßen Verfahrens wird der Randbereich des unfertigen Keramikkörpers aktiviert. Genauer gesagt werden die Atome in dem Randbereich des noch nicht erfindungsgemäß veränderten Keramikkörpers in einen energetisch angeregten Zustand versetzt. Dies ist erforderlich, um die Bildung des erfindungsgemäßen Mischoxid-Randbereichs zu ermöglichen.

Für die Randbereichaktivierung können erfindungsgemäß die aus dem Stand der Technik bekannten Verfahren angewendet werden, wie Beflammen mit einem Brenner, Plasmabehandlung, Coronabehandlung. Bevorzugt erfolgt die Randbereichaktivierung durch ein Plasmaverfahren.

Die Randbereichaktivierung mittels Plasma hat den Vorteil, dass die Oberfläche des Keramiksubstrats unter anderem zunächst gereinigt, d.h. von Verunreinigungen befreit wird. Bevorzugt ist eine Plasmabehandlung, bei der neben der Aktivierung des Randbereichs des Keramikkörpers zusätzlich der Randbereich im Sinne einer plasmachemischen Aktivierung angeätzt und aktiviert wird, um eine Vergrößerung der Reaktionsfläche und eine erhöhte Reaktionsbereitschaft für die erwünschte thermochemische Reaktion zwischen Metall (I) und Metall (II) zu schaffen. Die Reaktivität des Metalls (I) wird dadurch gesteigert.

Bevorzugt erfolgt die Randbereichaktivierung mit einem durch eine elektrische Gasentladung im Hochvakuum erzeugten Plasma, wobei die Energie und die Einwirkdauer des Plasmas auf die Oberfläche des Keramikkörpers so gewählt ist, dass die Atome des Randbereiches derart aktiviert werden, dass in dem Randbereich des Keramikkörpers eine chemische Reaktion überhaupt möglich wird und stattfinden kann.

Bevorzugt werden vor der Aktivierung des Randbereichs des Keramikkörpers die freigesetzten Verunreinigungen ausgegast. Das Ausgasen erfolgt vorzugsweise für mehrere Stunden, kann aber auch wesentlich kürzer oder länger erfolgen, bei einer Temperatur von 25°C bis 400°C, vorzugsweise unterhalb von 350°C, wobei andere Temperaturen nicht ausgeschlossen werden, und einem Druck von vorzugsweise 10⁻⁷ bis 10⁻³ mbar, wobei die Ausgasungen mittels Vakuumpumpen kontinuierlich aus der Reaktionskammer abgepumpt werden.

Der Randbereich des Werkstoffs oder Bauteils wird dann zur Aktivierung durch Ionen und/oder Elektronen, die durch eine elektrische Gasentladung im Hochvakuum erzeugt werden, beschossen. Der Druck in der Reaktionskammer liegt im Bereich zwischen 10⁻⁵ und 10⁻³ mbar, bevorzugt 10⁻⁷ und 10⁻³ mbar, besonders bevorzugt im Bereich des Weltraumvakuums. Bei diesen Drücken ist die mit der mittleren freien Weglänge korrelierte Energie der Plasmateilchen ausreichend groß, um die im Randbereich des Keramikkörpers befindlichen Atome energetisch derart anzuregen, dass im Randbereich des Keramikkörpers chemische Reaktionen möglich werden, die bei anderen Bedingungen nicht möglich sind.

Die Plasmaaktivierung wird nach den dem Fachmann bekannten Verfahren durchgeführt.

Als Gase für die Gasentladung werden Edelgase verwendet. Das Edelgas wird unter Argon, Neon, Krypton und Xenon ausgewählt, wobei Argon bevorzugt ist. Andere geeignete Edelgase sind nicht ausgeschlossen.

Es muss daher sichergestellt werden, dass die Metallionen (II) ungehindert, d.h. ohne auf dem Weg zwischen Target und Keramikkörper zu reagieren, auf den Keramikkörper auftreffen, um mit diesem thermochemisch und gleichmäßig reagieren zu können.

In Schritt (c) wird der Randbereich des unfertigen Keramikkörpers einer thermochemischen Behandlung unterworfen. Hierbei wird die chemische Zusammensetzung des Randbereichs des Keramikkörpers geändert.

Thermochemische Behandlung im Sinne dieser Erfindung ist eine Wärmebehandlung, die einem Werkstoff (Metall (I)) mit dem Ziel zugeführt wird, die chemische Zusammensetzung des Werkstoffes durch Stoffaustausch mit dem zugeführten Medium (Metall (II)) zu verändern. Im Allgemeinen werden bei der thermochemischen Behandlung metallische bzw. nichtmetallische Elemente in die Oberfläche eines Werkstoffs eindiffundiert. Im Zuge der thermochemischen Behandlung kann entweder ein Diffusionsbereich oder ein Verbindungsbereich mit darunter befindlichem Diffusionsbereich entstehen. Innerhalb eines Diffusionsbereichs fällt der Gehalt des eindiffundierten Elements (Metall II) kontinuierlich, gleichmäßig, allmählich zum Kern hin ab und der Gehalt des reagierenden Elements (Metall I) kontinuierlich, gleichmäßig, allmählich zur Oberfläche hin ab; dagegen ist der Konzentrationsabfall bei einem Verbindungsbereich in der Regel sehr steil.

Erfindungsgemäß erfolgt der Start der thermochemischen Reaktion des Keramikkörpers unter Zuhilfenahme der lonenimplantation. Dies bedeutet, dass in einer ersten Stufe Metall (II)-lonen in den Randbereich des unfertigen Keramikkörpers (Metall (I)) implantiert werden, von wo aus diese weiter in das Innere des Keramikkörpers diffundieren und reagieren können. Somit kommt es zur Ausbildung eines Verbindungsbereichs, nämlich im Bereich der lonenimplantation, und eines darunter liegenden Diffusionsbereichs. Durch die hohe Energie der Metall (II)-lonen und der in Schritt (b) erfolgten Randbereichaktivierung reagieren in der zweiten Stufe die Metall (II)-lonen mit den Sauerstoffatomen des Keramikmaterials (Metall (I)) unter Bildung eines Mischoxides (Metall (I)+(II)). Diese thermochemische Reaktion findet nur statt, wenn die Reaktionskammer zuvor im Sinne des Schritts (a) des erfindungsgemäßen Verfahrens evakuiert wurde. Bevorzugt erfolgt die lonenimplantation im Plasma. Besonders bevorzugt ist die lonenimplantation eine Plasmaimmersions-Ionenimplantation (PIII).

Die in dieser besonderen Art und Weise kombinierten Schritte ermöglichen überhaupt erst die thermochemische Reaktion von Reintitan und Oxidkeramik als Festkörper, damit ein monolithischer Keramikkörper mit einem Mischoxid-Randbereich und metallischer Oberfläche geschaffen werden kann.

Bei lonenimplantationsverfahren werden Ionen, die aus einem Target erzeugt werden, in einem gerichteten elektrischen Feld beschleunigt und treffen dann auf einen Festkörper. Die Ionen dringen in den Körper ein und bilden dabei eine oberflächliche Durchdringungsschicht. Die lonenimplantation kann durch die Parameter lonenenergie und lonendosis beeinflusst werden. Die lonenenergie bestimmt die Eindringtiefe, die lonendosis die Anzahl der implantierten Ionen. Mit Hilfe der Plasma-Immersions-Ionenimplantation (PIII) können die Vorteile der konventionellen lonenimplantation auf großflächige, komplex geformte Geometrien übertragen werden. Das zu behandelnde Werkstück wird dazu - erfindungsgemäß in einer Hochvakuumkammer - von einem durch eine geeignete Plasmaquelle generierten Plasma umhüllt; durch Anlegen von negativen Hochspannungspulsen mit sehr kurzen Pulsanstiegszeiten (< 1 Mikrosekunde) werden die beweglicheren Elektronen des Plasmas daraufhin zurückgestoßen und die zurückbleibenden positiven Ionen auf das Werkstück beschleunigt (implantiert). Die Beschleunigungsspannungen liegen dabei unterhalb der der herkömmlichen Ionenimplantation (Größenordnung: 30 kV). Da hierbei die gesamte Fläche gleichzeitig implantiert wird, ist dieses Verfahren gerade bei den in der Medizin anzutreffenden verschiedenen komplexen geometrischen Formen außerordentlich produktiv.

Als Target-Materialien können entsprechend den erwünschten Eigenschaften des erfindungsgemäßen monolithischen Keramikkörpers mit Mischoxid-Randbereich und metallischer Oberfläche alle dafür geeigneten Metalle und Legierungen verwendet werden. Diese werden hochenergetisch mit Magnetron, Laser, oder jedem anderen geeigneten Verfahren verdampft, um eine hohe "Dampfkonzentration" in der Hochvakuumkammer zu erzielen. Geeignete Target-Materialien umfassen Metalle mit einer hohen Sauerstoffaffinität. Vorzugsweise umfassen die Target-Materialien Ti, Nb, Ta, Legierungen davon/oder Verbindungen. Bevorzugte Materialien sind Titan, eine Titanverbindung oder eine Titanlegierung, wobei die Titanverbindung eine Verbindung aus Titan mit Elementen der 14. (z.B. C, Si, Ge, Sn, Pb), 15. (z.B. N, P, As, Sb, Bi) oder 16. (z.B. O, S, Se, Te, Po) Gruppe des Periodensystems oder ein Gemisch davon ist. Besonders bevorzugt sind elementares Titan und dessen Legierungen/Verbindungen, wobei elementares Titan ganz besonders bevorzugt ist.

Erfindungsgemäß wird für das Ablaufen der thermochemischen Reaktion in dem Randbereich die lonenimplantation oder die Plasmaimmersions-lonenimplantation mit einer lonendosis von 10¹⁵ bis 10¹⁶ lonen/cm² und einer lonenenergie von 1 MeV bis 2,3 MeV durchgeführt, unabdingbar in Kombination mit Hochvakuum. Die Temperatur beträgt zwischen Zimmertemperatur und 400 C, besonders bevorzugt 350 C und darunter. Der Druck beträgt etwa 10⁻³ bis etwa 10⁻⁷ mbar, besonders bevorzugt unter Weltraumatmosphäre.

Das Plasma kann kontinuierlich (cw-Plasma) oder gepulst erzeugt werden. Durch die Plasmaparameter, wie der Plasmapuls oder die Energie des Plasmapulses, können die Eigenschaften der Randzone, d.h. des resultierenden Mischoxid-Randbereichs und der resultierenden metallischen Oberfläche, eingestellt werden. Erfindungsgemäß kann entweder ein cw-Plasma oder ein gepulstes Plasma eingesetzt werden. Auch eine Kombination der beiden Plasmaerzeugungsarten ist möglich. Bevorzugt wird in Schritt (c) ein cw-Plasma eingesetzt, welches gegen Ende des Reaktionsprozesses in gepulst wechseln kann.

Die Erfinder haben überraschend festgestellt, dass die mit der herkömmlichen lonenimplantation einhergehenden Phänomene der Ion-Werkstoff-Wechselwirkung, wie Strahlenschädigung, Defektwechselwirkungen, Amorphisierung, Kristallisation, Ausscheidungsbildung, welche eine thermische Nachbehandlung (Tempern) erforderlich machen, nicht auftreten. Das Ziel der bisher für die Herstellung von Dentalimplantaten auf der Basis eines Titangrundkörpers und einer Keramikbeschichtung verwendeten lonenimplantations-Verfahren war die Reduktion der Sauerstoffaffinität des Titans während der Keramikbeschichtung (L. Wehnert, A. Moormann und W. Freesmeyer, Simulationsrechnungen zur Thermodynamik des konventionellen Titan-Keramik-Verbundes und zum Einfluss des verbundverbessernden lonenimplantationsverfahrens, Quintessenz Zahntech 1998, Band 24, Seite 1027-1037). Bei der vorliegenden Erfindung jedoch wird die hohe Sauerstoffaffinität des Metalls (II) ausgenutzt. Die Erfinder haben herausgefunden, dass die implantierten Metallionen (II) aufgrund der hohen Sauerstoffaffinität mit dem Sauerstoff der Keramik unter Bildung eines komplexen atomaren Verbundes reagieren. Der Randbereich des unfertigen Keramikkörpers wird hierdurch chemisch in eine Randzone verändert, d.h. ein Mischoxid aus Metall (I) und (II) wird gebildet, und es entsteht auf dem Mischoxid-Randbereich eine metallische Oberfläche aus dem Metall (II), wodurch die erwähnten Probleme der herkömmlichen lonenimplantation vermieden und ein anschließendes Tempern nicht mehr notwendig wird. Eine Schädigung der Keramik durch die Prozesse wird komplett vermieden, insbesondere auch durch die Wahl der relativ niedrigen Temperatur, und es entsteht eine monolithische Keramik mit Mischoxid-Randbereich und metallischer Oberfläche und nicht eine beschichtete Keramik. Das Fehlen von Phasengrenzen verdeutlicht den Unterscheid zur Beschichtung, und konsekutiv ist der erschaffene Keramikkörper ein Monolith und keine beschichtete Keramik. Die bisher ungelöste Schichthaftungsproblematik wird dadurch gelöst.

Die thermochemische Behandlung im Sinne des Schrittes (c) bewirkt, dass unter Hochvakuum hochenergetische Metall (II)-lonen (z.B. Titanionen) in den Randbereich des unfertigen Keramikkörpers eindringen und dort mit dem Sauerstoff des Metall (I)-Oxids (z.B. Zirkonoxid) ein komplexes Metall (I)-Metall (II)-Oxid (z.B. Titan-Zirkon-Oxid) sowie zusätzlich eine metallische Oberfläche aus dem Metall (II) bilden. Sie rufen somit eine chemische Reaktion hervor und wandeln den unfertigen Keramikkörper in seinem Randbereich derart in eine Randzone um, dass sich in letzterer das Metall (I) (z.B. Zirkon) und Sauerstoff auf atomarer Ebene mit den Metall (II)-Atomen (z.B. Titanatomen, Titanionen) verbinden und außerdem eine metallische Oberfläche aus dem Metall (II) ausbildet. Dementsprechend bildet das komplexe Metall (I)-Metall (II)-Oxid mit seiner metallischen Oberfläche keine Beschichtung, sondern stellt eine chemische Umwandlung des Randbereiches des unfertigen Keramikkörpers dar. Der Kern des Keramikkörpers und seine Randzone bilden daher eine monolithische Struktur, die in der metallischen Metall (II)-Oberfläche endet. Die Konzentration des ersten Metalls (I) und des weiteren Metalls (II) in dem Randbereich des erfindungsgemäßen Keramikkörpers beträgt idealerweise in der Mitte des Mischoxid-Randbereiches von (I) und (II) 50/50 %.

Vereinfacht formuliert kann man sagen, dass die thermochemische Reaktion den unfertigen Keramikkörper in einen neuen monolithischen Körper ohne Phasengrenzen umwandelt (Keramik im Kern, Mischoxid dazwischen, und Titan außen). Die Dicke ist je nach Bedarf und Anwendungsfall steuer- und regelbar.

In einer erfindungsgemäßen Ausführungsform kann der Keramikkörper mit dem Mischoxid-Randbereich und metallischer Oberfläche ferner mit einem oder mehreren Metallen, insbesondere dem weiteren Metall (II), beschichtet werden. Die Beschichtung mit einem oder mehreren Metallen erfolgt hierbei nach dem Fachmann bekannten und im Stand der Technik üblichen Verfahren zur Beschichtung von Metallen oder Keramiken.

In einer weiteren Ausführungsform kann die Beschichtung aus einem oder mehreren Metallen thermochemisch nitriert, boriert carburiert, nitrocarburiert, etc werden. Selbstverständlich kann auch die metallische Oberfläche des Monolithen aus dem Metall (II) gleich ohne weitere Beschichtung nitriert, boriert, carburiert, nitrocarburiert, etc. werden, falls benötigt (Gelenkflächen). Dies führt zur Härtung der metallischen Oberfläche des Keramikkörpers und erfolgt beispielsweise durch plasmagestützte thermochemische Nitrierung, Borierung, Carburierung, Nitrocarburierung, etc.

In einer weiteren erfindungsgemäßen Ausführungsform kann die Oberfläche des Keramikkörpers mit einem Mischoxid-Randbereich und metallischer Oberfläche mit einem oben beschriebenen biokompatiblen/bioaktiven Material beschichtet werden. Die Beschichtung mit dem biokompatiblen/bioaktiven Material erfolgt hierbei ebenfalls nach dem Fachmann bekannten und im Stand der Technik üblichen Verfahren zur Beschichtung von Keramiken oder Metallen.

Die vorliegende Erfindung betrifft auch die Verwendung des Keramikkörpers mit einem Mischoxid-Randbereich und metallischer Oberfläche als medizinisches Implantat, insbesondere als Zahnimplantat. Implantate können, je nach Anwendung, vollständig oder nur teilweise mit einem Mischoxid-Randbereich und metallischer Oberfläche versehen werden. "Teilweise" ist dahingehend zu verstehen, dass die Implantatbereiche, welche Knochenkontakt haben, soweit einen Mischoxid-Randbereich und eine metallische Oberfläche aufweisen, dass eine gesicherte Osseointegration gewährleistet ist.

"Medizinisch" im Sinne der Erfindung betrifft die Bereiche der Humanmedizin, einschließlich der Zahnmedizin, der Tiermedizin, einschließlich des dentalen Bereichs. Ein medizinisches Implantat im Sinne der Erfindung ist eine medizinische Vorrichtung, die dem Ersatz biologischer Strukturen im menschlichen oder tierischen Körper dient, oder in den Körper für andere Zwecke eingesetzt wird. Daher umfassen die medizinischen Implantate im Sinne der Erfindung Implantate und Zahnimplantate für den Menschen und für Tiere. Als medizinische Implantate sind Zahnimplantate, Hüftimplantate, Epithesen, künstliche Gelenke und Prothesen bevorzugt.

Eine Prothese ist eine künstliche Gliedmaße, die ein fehlendes Körperteil ersetzt (z.B. aufgrund Krankheit, Unfall oder Amputation), wohingegen eine Epithese primär kosmetische Funktion hat (z.B. als künstliches Auge oder Ohr). Medizinische Implantate und insbesondere Prothesen können verwendet werden um biologische Strukturen, wie Knochen, Gelenke oder Knochenteile, in nahezu allen Bereichen des Körpers, z.B. Schädel, Zähne, Ober- und Unterarm, Ellenbogen, Ober- und Unterschenkel, Hüfte, Zehen, Finger, Knie, Wirbelsäule, etc. zu ersetzen. Aber auch Hörhilfen, künstliche Gliedmaßen, Ersatzgelenke, und Haarprothesen (Perücken) sowie Implantate zur deren Befestigung fallen unter medizinische Implantate im Sinne der Erfindung. In speziellen Ausführungsformen können Hörhilfen in andere Implantate integriert werden. Dies gilt auch für in den Körper implantierte "Heilmittel" oder deren Behältnisse (z.B Herzschrittmacher, Insulinpumpen, etc.).

Implantate und Zahnimplantate sind in einigen erfindungsgemäßen Ausführungsformen ein oder mehrteilige Implantate.

In einer bevorzugten Ausführungsform der Erfindung weist nur der Bereich des Keramikkörpers, der mit dem Knochen in Kontakt kommt, einen Mischoxid-Randbereich mit metallischer Oberfläche (vollständig oder partiell) auf. In einer anderen Ausführungsform weist zusätzlich der Bereich einen Mischoxid-Randbereich mit metallischer Oberfläche auf, der mit dem zweiten Teil eines zweiteiligen Implantats in Berührung kommt.

Ein Zahnimplantat ist insbesondere ein ein-, zwei- oder mehrteiliges Implantat und kann ein Schraubengewinde umfassen. Bevorzugt umfasst das Zahnimplantat einen Verankerungsteil, um das Implantat in dem Knochen zu verankern, und einen Befestigungsteil zur Aufnahme der Suprakonstruktion, wobei nur der Verankerungsteil einen Mischoxid-Randbereich aufweist. In einer speziellen Ausführungsform des zweiteiligen Implantats weist der Bereich des Keramikkörpers der mit dem anderen Bereich in Kontakt kommt (z.B. das Abutment an seiner Kontaktfläche zwischen dem Implantat und dem Abutment), einen partiellen Mischoxid-Randbereich mit metallischer Oberfläche auf. In diesem Fall ist keine Schraubenverbindung zwischen den beiden Teilen erforderlich, da eine optimale Passgenauigkeit erreicht werden kann, die zu einem guten Sitz und hoher Stabilität der Verbindung führt (Presspassung). Falls eine Schraube, für ein mehrteiliges Implantat erfindungsgemäß hergestellt wird (z.B. mit dem Implantat verschraubte Abutments), kann entweder die ganze Schraube, oder auch nur der Gewindebereich einen Mischoxid-Randbereich mit metallischer Oberfläche aufweisen.

In Fällen, in denen das Implantat mehrteilig ist, kann entweder nur ein Teil oder können beide Teile im Kontaktbereich der beiden Teile einen Mischoxid-Randbereich mit metallischer Oberfläche aufweisen. Ein Beispiel für eine derartige Ausführungsform ist ein künstliches Hüftgelenk. Dabei kann mindestens ein Teil des künstlichen Gelenks einen Mischoxid-Randbereich mit metallischer Oberfläche aufweisen. Zum Beispiel weist neben dem Bereich, der mit dem Knochen in Verbindung kommt, auch der Bereich des Hüftgelenks einen Mischoxid-Randbereich mit metallischer Oberfläche auf, der mit dem Kopf (Kugel) in Berührung kommt. Oder, umgekehrt weist nur der Bereich, der mit der Pfanne in Berührung kommt, einen Mischoxid-Randbereich mit metallischer Oberfläche auf. Es ist auch denkbar beide Implantatteile erfindungsgemäß vollständig mit Mischoxid-Randbereich und metallischer Oberfläche zu versehen, damit die gefürchtete Splitterwirkung beim Bruch des Implantates weitestgehend verhindert wird. Ein Vorteil wäre, dass ein Mischoxid-Randbereich mit metallischer Oberfläche das quietschende oder unerwünschte Geräusch, das während der Bewegung der Gelenke auftreten kann, verhindert. Insbesondere ein Mischoxid-Randbereich mit metallischer Oberfläche in dem Kopfbereich eines künstlichen Hüftgelenks kann quietschende Geräusche verhindern und dient als "Schmiermittel".

Der erfindungsgemäße monolithische Keramikkörper mit Mischoxid-Randbereich und metallischer Oberfläche kann in einigen Ausführungsformen eine zusätzliche Schicht aus diamantähnlicher Kohlenstoffschicht (DLC) umfassen (z.B. an Gelenkflächen). DLC ist eine amorphe extrem harte Kohlenstoffschicht. In einigen Ausführungsformen kann die Zusammensetzung ein oder mehrere weitere Metallschichten umfassen, wie Gold, Silber, Platin, Aluminium, Kupfer, Eisen, Nickel, Zinn, Tantal, Zink und/oder Chrom, und/oder Legierungen, wie Stahl oder Bronze.

### Figurenbeschreibung

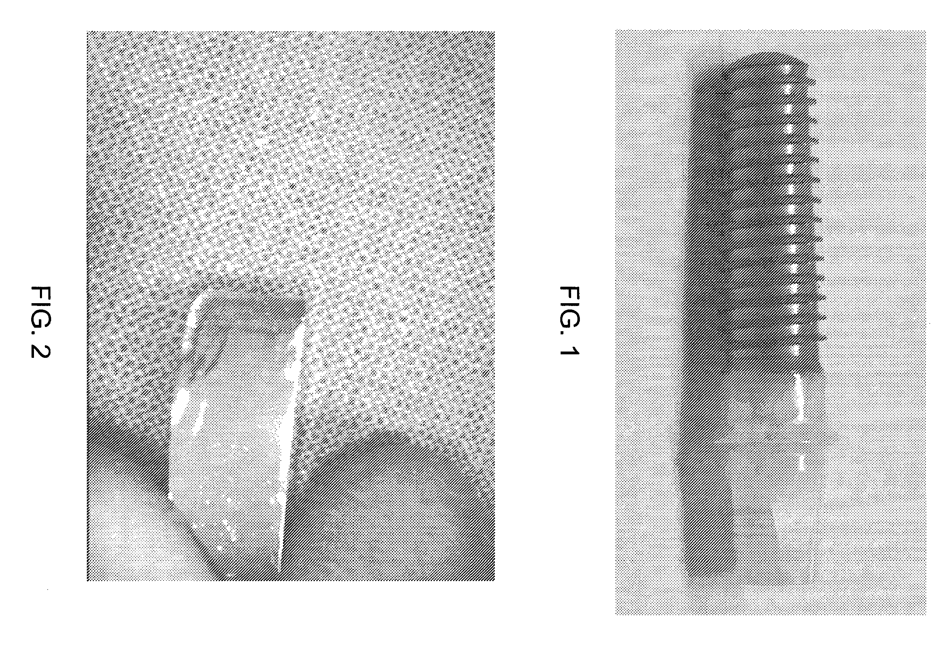

- FIG. 1:: zeigt einen erfindungsgemäßen Keramikkörper in Form eines einteiligen Zahnimplantats. Nur der mit Gewinde versehene Verankerungsteil zum Verankern des Implantats im Knochen weist einen Zirkonia-Alumina-Titan-Mischoxid-Randbereich, mit metallischer Oberfläche aus Reintitan auf. Der Kern des Zahnimplantats besteht aus Zirkonia-Alumina Keramik.
- FIG. 2:: zeigt einen gebrochenen erfindungsgemäßen Keramikkörper mit Kern aus Zirkonia-Alumina-Keramik, Mischoxid-Randbereich aus Zirkonia-Alumina- und Titan-Mischoxid und metallischer Oberfläche aus Reintitan. Der erfindungsgemäße Vorteil der mangelnden Zersplitterneigung ist gut zu erkennen.
- FIG. 3:: zeigt die REM-Aufnahme einer Bruchstelle eines erfindungsgemäßen Keramikkörpers mit Kern aus Alumina-Keramik, Mischoxid-Randbereich aus Alumina-Titan-Mischoxid und metallischer Oberfläche aus Reintitan. Im Hintergrund ist die metallische Oberfläche zu erkennen, die hier eine "weiche" knochenähnliche Struktur aufweist und Mikrofrakturen im Implantatbett vermeiden soll. Die blanke Fläche im Vordergrund ist der Mischoxid-Randbereich sowie der Alumina-Kern.
- FIG. 4:: zeigt zwei ca. 1 mm dicke Oxidkeramikplättchen, links erfindungsgemäß mit metallischer Oberfläche aus Titan und rechts herkömmlich ohne Titan-Oberfläche. Das erfindungsgemäße Keramikplättchen weist einen Kern aus Zirkonia-Alumina-Keramik und einen Mischoxid-Randbereich aus Zirkonia-Alumina-Titan-Mischoxid sowie eine metallische Oberfläche aus Reintitan auf.
- FIG. 5a, 5b:: jeweils zwei definierte Bruchstücke des erfindungsgemäßen Keramikplättchens aus Fig. 4. Eine Zersplitterung in viele Einzelteile trat beim Bruchtest nicht auf. Während des Biegens bis hin zum Bruch gab es auch keine Abplatztendenzen der Oberfläche, wie bei herkömmlichen Beschichtungen üblich.

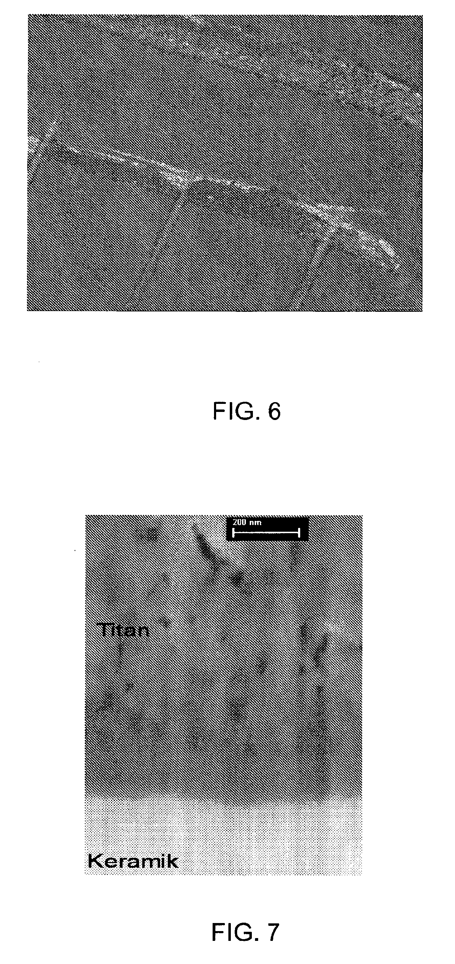

- FIG. 6:: zeigt eine Gitterschnittprüfung eines erfindungsgemäßen Keramikkörpers mit Kern aus Aluminiumoxid-Keramik, Mischoxid-Randbereich aus Aluminiumoxid-Titan-Mischoxid und metallischer Oberfläche aus Reintitan. Es sind keine Abplatztendenzen zu erkennen. Die den Kern bildende Keramik liegt nicht frei.
- FIG. 7:: zeigt die REM-Querschnitts-Aufnahme (starke Vergrößerung) eines erfindungsgemäßen Keramikkörpers mit Kern aus Zirkonia-Alumina-Keramik, Mischoxid-Randbereich aus Zirkonia-Alumina-Titan-Mischoxid und metallischer Oberfläche aus Reintitan. Im unteren Bereich ist hell der Keramikkern zu erkennen. Daran schließt sich nach oben der gräuliche Mischoxid-Randbereich (ca. 700 Atomlagen) an, auf dem sich die ungleichmäßig dunkelgrau-schwarze Oberfläche aus Reintitan befindet.

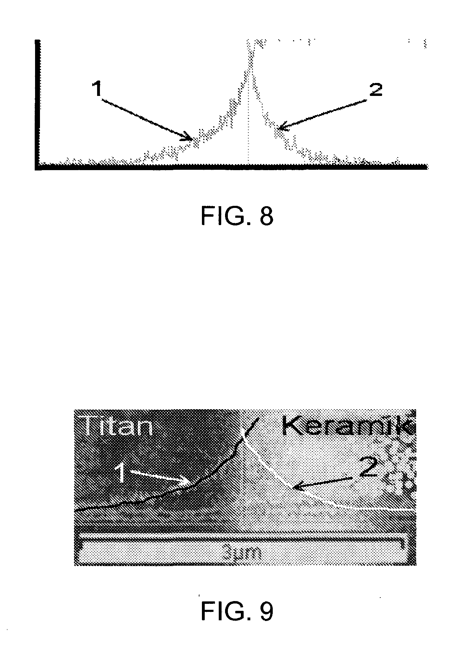

- FIG. 8:: zeigt ein EDX-Diagramm betreffend den Keramikkörper aus FIG. 7. Es zeigt die Konzentrationsverläufe der Metalle (I) und (II) im Mischoxid-Randbereich. Auf der y-Achse ist beginnend mit 0 % und in Richtung Diagrammoberkante ansteigend die Konzentration des jeweiligen Metalls aufgetragen. Auf der x-Achse ist die in Richtung des Kerns des Keramikkörpers verlaufende Tiefenkoordinate aufgetragen, wobei der Abszissenwert x = 0 in dem Übergangsbereich zwischen dem Mischoxid-Randbereich und der metallischen Oberfläche liegt; Kurve 1 (Zirkonia-Alumina) zeigt die Metall (I) Konzentration, welche in Richtung Oberfläche (nach links) gegen 0% läuft; Kurve 2 (Titan) zeigt die Metall (II) Konzentration, welche in Richtung Kern gegen 0% läuft; Die aus den beiden Kurven entstandene zeltförmige Struktur zeigt den erwünschten Effekt der Konzentrationen von 50/50 % der Metalle (I)/(II) in der Mitte des Mischoxid-Randbereiches. Es ist zu erkennen, dass die beiden Kurven in Richtung Mitte des Mischoxid-Randbereichs gleichmäßig gegeneinander und von der Mitte des Mischoxid-Randbereichs weg gleichmäßig auseinander verlaufen
- FIG. 9:: zeigt das EDX-Diagramm gemäß FIG. 8, welches in die REM-Querschnitts-Aufnahme gemäß FIG. 7 an der entsprechenden Stelle hineinprojiziert ist. Es gilt analog das zu FIG. 8 Gesagte.

## Patentansprüche

1. Monolithischer Keramikkörper mit Mischoxid-Randbereich und metallischer Oberfläche, wobei der Keramikkörper einen Kern aus dem Oxid eines ersten Metalls (I) sowie eine Randzone, die aus einem Mischoxid-Randbereich, welcher das Oxid des ersten Metalls (I) und das Oxid eines weiteren Metalls (II) umfasst, welches eine hohe Affinität zu Sauerstoff hat, und aus einer auf dem Mischoxid-Randbereich befindlichen metallischen Oberfläche aus dem Metall (II) besteht, umfasst,
wobei die Randzone durch Änderung der chemischen Zusammensetzung eines Randbereiches eines unfertigen Keramikkörpers mittels einer Aktivierung des Randbereiches und einer sich anschließenden thermochemischen Behandlung jeweils unter einem Unterdruck, der 10⁻³ mbar oder weniger beträgt, entstanden ist und sich die Änderung der chemischen Zusammensetzung dadurch auszeichnet, dass es nicht nur zu einem Einbau von Ionen des Metalls (II) in das Gitter des Keramikmaterials des Keramikkörpers gekommen ist,
wobei die thermochemische Behandlung durch lonenimplantation eingeleitet wurde,
wobei der Mischoxid-Randbereich
einen kontinuierlichen Konzentrationsgradienten des ersten Metalls (I), ausgehend von 100% im Kern bis zu 0% im Übergangsbereich zu der metallischen Oberfläche des Keramikkörpers, bezogen auf den Gesamtmetallgehalt (I+II), und
einen kontinuierlichen Konzentrationsgradienten des weiteren Metalls (II), ausgehend von 0% im Kern bis zu 100% im Übergangsbereich zu der metallischen Oberfläche des Keramikkörpers, bezogen auf den Gesamtmetallgehalt (I+II),
aufweist,
wobei die Sauerstoffkonzentration in dem Mischoxid-Randbereich konstant bleibt, und
wobei die monolithische Struktur des Keramikkörpers phasengrenzenlos ausgebildet ist.

2. Der Keramikkörper nach Anspruch 1, worin das erste Metall (I) unter Aluminium, Zirkonium, Yttrium, Niobium, Hafnium, Silizium, Magnesium, Cer oder Mischformen der genannten Metalle ausgewählt ist.

3. Der Keramikkörper nach Anspruch 2, worin das erste Metall (I) Zirkonium oder Aluminium oder eine Zirkonium-Aluminium-Mischung ist.

4. Der Keramikkörper nach einem der vorhergehenden Ansprüche, worin das weitere Metall (II) biokompatibel ist.

5. Der Keramikkörper nach Anspruch 4, worin das biokompatible Metall (II) Titan ist.

6. Der Keramikkörper nach Anspruch 3 und 5, worin der Mischoxid-Randbereich von einem Titan-Zirkonium-Mischoxid, Titan-Alumina-Mischoxid oder Titan-Alumina-Zirkonia-Mischoxid gebildet wird, und die metallische Oberfläche aus Reintitan besteht.

7. Der Keramikkörper nach einem der vorhergehenden Ansprüche, worin eine Randzone des Keramikkörpers umfassend den Mischoxid-Randbereich und die darauf befindliche metallische Oberfläche zwischen 0,05 und 140 µm dick ist.

8. Der Keramikkörper nach einem der vorhergehenden Ansprüche, welcher ferner eine oder mehrere Schichten aus weiteren Metallen, insbesondere dem Metall (II), umfasst.

9. Der Keramikkörper nach einem der vorhergehenden Ansprüche, welcher zusätzlich eine oder mehrere biokompatible und/oder bioaktive Beschichtungen umfasst.

10. Verfahren zur Herstellung eines Keramikkörpers mit einem Mischoxid-Randbereich mit metallischer Oberfläche nach einem der Ansprüche 1 bis 9, welches folgende in einer thermochemischen Reaktionskammer an einem unfertigen Keramikkörper mit einem Randbereich in folgender Reihenfolge durchzuführende Schritte umfasst:
(a) Evakuierung der Reaktionskammer auf einen Unterdruck, der 10⁻³ mbar oder weniger beträgt,
(b) Aktivierung des Randbereichs des unfertigen Keramikkörpers unter dem in Schritt (a) erzeugten Unterdruck, und
(c) Änderung der chemischen Zusammensetzung des Randbereiches des unfertigen Keramikkörpers durch eine thermochemische Behandlung unter dem in Schritt (a) erzeugten Unterdruck derart, dass der Randbereich chemisch in eine Randzone des fertigen Keramikkörpers umgewandelt wird, welche aus der metallischen Oberfläche und dem darunter liegenden Mischoxid-Randbereich besteht, der unterhalb der metallischen Oberfläche beginnt, wobei sich die Änderung der chemischen Zusammensetzung dadurch auszeichnet, dass es nicht nur zu einem Einbau von Ionen des Metalls (II) in das Gitter des Keramikmaterials des Keramikkörpers kommt und wobei die thermochemische Behandlung durch lonenimplantation mit einer Ionendosis von 10¹⁵ bis 10¹⁶ Ionen/cm² und einer lonenenergie von bis zu 2,3 MeV eingeleitet wird.

11. Das Verfahren nach Anspruch 10, wobei die Oberflächenaktivierung in Schritt (b) mittels einer Plasmabehandlung erfolgt.

12. Das Verfahren nach Anspruch 10, wobei die lonenimplantation eine Plasmaimmersions-lonenimplantation ist.

13. Das Verfahren nach einem der vorhergehenden Ansprüche 10 bis 12, wobei Schritt (c) bei einer Temperatur von 20° bis 400°C durchgeführt wird.

14. Das Verfahren nach einem der vorhergehenden Ansprüche 10 bis 13, wobei das Verfahren den zusätzlichen Schritt (d) des Beschichtens der Oberfläche des Keramikkörpers mit einem oder mehreren Metallen, insbesondere dem Metall (II), umfasst.

15. Das Verfahren nach einem der vorhergehenden Ansprüche 10 bis 14, wobei das Verfahren ferner den Schritt (e) des Beschichtens der Oberfläche des Keramikkörpers mit einem biokompatiblen und/oder bioaktiven Material umfasst.

16. Das Verfahren nach einem der vorhergehenden Ansprüche 10 bis 15, wobei der Mischoxid-Randbereich mit metallischer Oberfläche nur in einem Teilbereich des unfertigen Keramikkörpers ausgebildet wird.

17. Verwendung des Keramikkörpers nach einem der Ansprüche 1 bis 9 oder des nach einem der Ansprüche 10 bis 16 hergestellten Keramikkörpers als Implantat.

18. Verwendung des Keramikkörpers nach einem der Ansprüche 1 bis 9 oder des nach einem der Ansprüche 10 bis 16 hergestellten Keramikkörpers als Schutzpanzerung für Personen oder Landfahrzeuge oder Luftfahrzeuge oder Wasserfahrzeuge oder Gebäude oder Raumfahrzeuge.

## Claims

1. A monolithic ceramic body with mixed-oxide marginal region and metallic surface, wherein the ceramic body has a core of the oxide of a first metal (I) and a marginal zone, the marginal zone comprising a mixed-oxide marginal region, which comprises the oxide of the first metal (I) and the oxide of another metal (II), which has a high affinity for oxygen, and a metallic surface of the metal (II) on the mixed-oxide marginal region,
wherein the marginal zone has been formed by altering the chemical composition of a marginal region of an unfinished ceramic body by means of an activation of the marginal region and a subsequent thermochemical treatment each under a negative pressure that is 10⁻³ mbar or less, and wherein the altering of the chemical composition distinguishes in that it results not only in an incorporation of ions of the metal (II) into the lattice of the ceramic material of the ceramic body,
wherein the thermochemical treatment has been induced by ion implantation,
wherein the mixed-oxide marginal region
has a continuous concentration gradient of the first metal (I), starting from 100% in the core to 0% in the transition region to the metallic surface of the ceramic body, relative to the total metal content (I+II), and
has a continuous concentration gradient of the another metal (II), starting from 0% in the core to 100% in the transition region to the metallic surface of the ceramic body, relative to the total metal content (I+II),
wherein the oxygen concentration of the mixed-oxide marginal region remains constant, and
wherein the monolithic structure of the ceramic body is formed without phase boundaries.

2. The ceramic body as claimed in claim 1, in which the first metal (I) is selected from aluminum, zirconium, yttrium, niobium, hafnium, silicon, magnesium, cerium or mixed forms of the stated metals.

3. The ceramic body as claimed in claim 2, in which the first metal (I) is zirconia or alumina or a zirconia-alumina mixture.

4. The ceramic body as claimed in one of the preceding claims, in which the another metal (II) is biocompatible.

5. The ceramic body as claimed in claim 4, in which the biocompatible metal (II) is titanium.

6. The ceramic body as claimed in claim 3 and 5, in which the mixed-oxide marginal region is formed by a titanium-zirconium mixed oxide, titanium-alumina mixed oxide or titanium-alumina-zirconia mixed oxide, and the metallic surface consists of pure titanium.

7. The ceramic body as claimed in one of the preceding claims, in which a marginal zone of the ceramic body comprising the mixed-oxide marginal region and the metallic surface thereon is between 0.05 and 140 µm thick.

8. The ceramic body as claimed in one of the preceding claims, which further comprises one or more layers of further metals, especially metal (II).

9. The ceramic body as claimed in one of the preceding claims, which additionally comprises one or more biocompatible and/or bioactive coatings.

10. A method of producing a ceramic body with a mixed-oxide marginal region with metallic surface as claimed in one of claims 1 to 9, which comprises the following steps to be carried out in the following sequence in a thermochemical reaction chamber on an unfinished ceramic body with a marginal region:
(a) evacuating the reaction chamber to a negative pressure that is 10⁻³ mbar or less,
(b) activating the marginal region of the unfinished ceramic body under the negative pressure generated in step (a), and
(c) altering the chemical composition of the marginal region of the unfinished ceramic body by means of a thermochemical treatment under the negative pressure generated in step (a) in such manner that the marginal region is chemically transformed into a marginal zone of the finished ceramic body, the marginal zone comprising the metallic surface and the mixed-oxide marginal region thereunder which begins underneath the metallic surface, wherein the altering of the chemical composition distinguishes in that it results not only in an incorporation of ions of the metal (II) into the lattice of the ceramic material of the ceramic body, and wherein the thermochemical treatment is induced by ion implantation having an ion dose from 10¹⁵ to 10¹⁶ ions/cm² and an ion energy of up to 2,3 MeV.

11. The method as claimed in claim 10, wherein the surface activation in step (b) takes place by a plasma treatment.

12. The method as claimed in claim 10, wherein the ion implantation is a plasma-immersion ion implantation.

13. The method as claimed in one of the preceding claims 10 to 12, wherein step (c) is carried out at a temperature from 20° to 400°C.

14. The method as claimed in one of the preceding claims 10 to 13, wherein the method comprises the additional step (d) of coating the surface of the ceramic body with one or more metals, especially metal (II).

15. The method as claimed in one of the preceding claims 10 to 14, wherein the method further comprises the step (e) of coating the surface of the ceramic body with a biocompatible and/or bioactive material.

16. The method as claimed in one of the preceding claims 10 to 15, wherein the mixed-oxide marginal region with metallic surface is only formed in a partial region of the unfinished ceramic body.

17. The use of the ceramic body as claimed in one of the preceding claims 1 to 9 or of the ceramic body produced as claimed in one of the preceding claims 10 to 16 as implant.

18. The use of the ceramic body as claimed in one of the preceding claims 1 to 9 or of the ceramic body produced as claimed in one of the preceding claims 10 to 16 as protective armour plating for persons or land vehicles or aircraft or watercraft or buildings or spacecraft.

## Revendications

1. Corps céramique monolithique avec une partie périphérique en mélange d'oxydes et une surface métallique, le corps céramique comprenant un noyau en un oxyde d'un premier métal (I), ainsi qu'une zone périphérique constituée d'une partie périphérique en mélange d'oxydes, qui comprend l'oxyde du premier métal (I) et l'oxyde d'un autre métal (II) présentant une haute affinité pour l'oxygène, et d'une surface métallique en métal (II), située sur la partie périphérique en mélange d'oxydes,
la zone périphérique étant obtenue par modification de la composition chimique d'une partie périphérique d'un corps céramique non fini au moyen d'une activation de la partie périphérique et d'un traitement thermochimique qui suit, respectivement sous vide, à une sous-pression de 10³ mbar ou moins, et la modification de la composition chimique étant **caractérisée en ce qu'**il ne s'est pas seulement produit une incorporation d'ions du métal (II) dans le réseau du matériau céramique du corps céramique,
le traitement thermochimique ayant été déclenché par implantation ionique,
la partie périphérique en mélanges d'oxydes présente
un gradient continu de concentration du premier métal (I) allant de 100% dans le noyau à 0% dans la zone de transition vers la surface métallique du corps céramique, par rapport à la teneur totale en métal (I+II), et
un gradient continu de concentration de l'autre métal (II) allant de 0% dans le noyau à 100% dans la zone de transition vers la surface métallique du corps céramique par rapport à la teneur totale en métal (I+II),
la concentration en oxygène restant constante dans la partie périphérique en mélange d'oxydes et
la structure monolithique du corps céramique étant conçue sans phase limite.

2. Corps céramique suivant la revendication 1, dans lequel le premier métal (I) est sélectionné parmi le groupe constitué d'aluminium, de zirconium, d'yttrium, de niobium, d'hafnium, de silicium, de magnésium, de cérium ou d'un mélange des métaux cités.

3. Corps céramique suivant la revendication 2, dans lequel le premier métal (I) est du zirconium ou de l'aluminium ou un mélange de zirconium et d'aluminium.

4. Corps céramique suivant une des revendications précédentes, dans lequel l'autre métal (II) est biocompatible.

5. Corps céramique suivant la revendication 4, dans lequel le métal biocompatible (II) est du titane.

6. Corps céramique suivant les revendications 3 et 5, dans lequel la partie périphérique en mélange d'oxydes est formée par un mélange d'oxydes de titane-zirconium, un mélange d'oxydes de titane-alumine ou un mélange d'oxydes de titane-alumine-zircone et la surface métallique est en titane pure.

7. Corps céramique suivant une des revendications précédentes, dans lequel une zone périphérique du corps céramique, qui comprend la partie périphérique en mélange d'oxydes et la surface métallique située sur cette dernière, présente une épaisseur comprise entre 0,05 et 140µm.

8. Corps céramique suivant une des revendications précédentes comprenant, en outre, une ou plusieurs couches en d'autres matériaux, en particulier en métal (II).

9. Corps céramique suivant une des revendications précédentes comprenant, en outre, un ou plusieurs revêtements biocompatibles et/ou bioactifs.

10. Procédé de fabrication d'un corps céramique avec une partie périphérique en mélange d'oxydes avec une surface métallique suivant une des revendications 1 à 9, procédé qui comprend les étapes suivantes à exécuter dans l'ordre suivant, dans une chambre de réaction thermochimique sur un corps céramique non fini avec une partie périphérique :
(a) mise sous vide de la chambre de réaction jusqu'à une sous-pression de 10⁻³ mbar ou moins,
(b) activation de la partie périphérique du corps céramique non fini sous le vide produit à l'étape (a),
(c) modification de la composition chimique de la partie périphérique du corps céramique non fini par un traitement thermochimique sous le vide produit à l'étape (a), de sorte que la partie périphérique est chimiquement transformée en une zone périphérique du corps céramique fini, qui est constituée de la surface métallique et de la partie périphérique en mélange d'oxydes, située sous cette dernière, partie périphérique, qui commence sous la surface métallique, la modification de la composition chimique étant **caractérisée en ce qu'**il ne se produit pas seulement une incorporation d'ions du métal (II) dans le réseau du matériau céramique du corps céramique et que le traitement thermochimique est déclenché par implantation ionique avec une dose d'ions comprise entre 10¹⁵ à 10¹⁶ ions/cm² et une énergie de la liaison ionique jusqu'à 2,3 MeV.

11. Procédé suivant la revendication 10, l'activation de la surface ayant lieu à l'étape (b) au moyen d'un traitement plasma.

12. Procédé suivant la revendication 10, l'implantation ionique étant une implantation ionique par immersion plasma.

13. Procédé suivant une des revendications précédentes 10 à 12, l'étape ( c) étant exécutée à une température comprise entre 20° et 400°C.

14. Procédé suivant une des revendications précédentes 10 à 13, le procédé comprenant l'étape supplémentaire (d) consistant à revêtir la surface du corps céramique d'un ou de plusieurs métaux, en particulier du métal (II).

15. Procédé suivant une des revendications 10 à 14, le procédé comprenant, en outre, l'étape (e ) consistant à revêtir la surface du corps céramique d'un matériau biocompatible et/ou bioactif.

16. Procédé suivant une des revendications précédentes 10 à 15, la partie périphérique en mélange d'oxydes à surface métallique n'est réalisée que dans une zone partielle du corps céramique non fini.

17. Utilisation du corps céramique suivant une des revendications 1 à 9 ou du corps céramique fabriqué suivant une des revendications 10 à 16 comme implant.

18. Utilisation du corps céramique suivant une des revendications 1 à 9 ou du corps céramique fabriqué suivant les revendications 10 à 16 comme blindage pour des personnes ou des véhicules terrestres ou des aéronefs ou des bateaux ou des engins spatiaux.
